# EUROPEAN PATENT SPECIFICATION

(11) **EP 0 515 817 B1**
(45) Date of publication and mention of the grant of the patent: **18.12.1996**
(21) Application number: 92106416.8
(22) Date of filing: 14.04.1992
(51) Int. Cl.: G01N 25/20, G01N 33/38

(54) **Method of evaluating ceramics**
Verfahren zur Bewertung von Keramik
Procédé pour l'évaluation de céramique

(30) Priority: 16.04.1991 JP 110972/91
(43) Date of publication of application: 02.12.1992
(73) Proprietor: Sumitomo Electric Industries, Ltd., Osaka 541 (JP)
(72) Inventor: Yamakawa, Akira, c/o Itami Works of Sumitomo, Itami-shi, Hyogo (JP); Miyake, Masaya, c/o Itami Works of Sumitomo, Itami-shi, Hyogo (JP); Ishizaki, Kozo, Nagaoka-shi, Niigata-ken (JP); Watari, Koji, Nagoya-shi, Aichi-ken (JP)
(74) Representative: Grosse, Wolfgang, Dipl.-Ing.

(56) References cited:
- EP-A- 0 129 419
- DD-A- 279 802
- SOVIET INVENTIONS ILLUSTRATED, EL section, week 8605, March 12, 1986 DERWENT PUBLICATIONS LTD., London, S 03

## Description

The present invention relates to a method of evaluating ceramics according to claim 1. More particularly, it relates to a method of evaluating properties of ceramics by nondestructive means, more specifically, a method of evaluating mechanical properties of ceramics such as a creep property.

The properties of ceramics change greatly according to its manufacture process. It is thus indispensable to evaluate the properties of ceramics as each product obtained after the manufacture. Conventionally, numerous methods for evaluating the properties of ceramics in either a destructive state or nondestructive state have been considered. A description will now be made on a silicon nitride (Si₃N₄) as one example of ceramics.

Silicon nitride is noted as one of engineering ceramics to be used for structural members provided at high temperatures such as automobile engine components. Since sintering of silicon nitride alone is hard, however, a sintering aid made of a low-melting point compound is generally added to silicon nitride powder for sintering. As such a sintering aid, oxides such as of aluminum, magnesium, yttrium, lanthanum, cerium, beryllium or zirconium are generally employed alone or in combination.

A sintered body of silicon nitride with a higher density can be achieved by use of the sintering aid; however, the sintering aid remains as a glass phase or a crystal phase of a lower strength (hereinafter referred to as "glass phase or the like") in a grain boundary of a resultant sintered body. It is thus difficult to obtain a silicon nitride sintered body having such a mechanical property as a desired strength. Also, when the silicon nitride sintered body is manufactured by pressure sintering such as a Hot Press method or an HIP method, it is possible to reduce or eliminate the amount of the sintering aid to be added. However, the manufacture of the silicon nitride sintered body by such pressure sintering has the disadvantage that it is difficult to successively carry out sintering steps, resulting in an increase in manufacturing cost.

Accordingly, at present, a silicon nitride sintered body which is obtained industrially includes glass phase or the like in its grain boundary. It is considered in general that mechanical properties such as strength and in ceramics such as silicon nitride become deteriorated with an increase in amount of glass phase or the like included in the ceramics. It is extremely significant to precisely measure the amount of glass phase or the like included in the ceramics, in order to manufacture ceramics such as silicon nitride having excellent mechanical properties such as strength. However, a nondestructive inspection has been unknown which is introduced into a production line of ceramics and in which products flowing on the line are directly inspected and the amount of glass phase or the like can be measured.

That is to say, conventionally known methods of measuring the amount of glass phase or the like included in ceramics such as silicon nitride are only as follows: a structure observation method for observing structure or texture of a sintered body having abraded surfaces by employing a microscope or the like; an X-ray diffraction method for photographing an X-ray diffraction image of a sintered body which is controlled to have a predetermined thickness; a method for processing a sintered body in a predetermined shape and measuring internal friction as disclosed in Japanese Patent Laying-Open No. 63-1955; and the like. In accordance with those conventional methods, it is impossible to measure the amount of glass phase or the like included in ceramics in a nondestructive state, and hence, only a sampling inspection can be carried out on the production line. Thus, there has been no guarantee for the amount of glass phase or the like in ceramics to be supplied as products in practice.

The object of the present invention is to provide a method introduced into a production line and for evaluating individual material properties of ceramics and mechanical properties of ceramics by nondestructive means.

A further object of the present invention is to provide a method of evaluating a creep property of ceramics by nondestructive means.

A still further object of the present invention is to provide a method of evaluating mechanical properties of a silicon nitride sintered body by nondestructive means.

In accordance with a method of evaluating ceramics according to one aspect of the present invention, a specific heat of ceramics to be an object of evaluation is measured at a predetermined temperature. Comparison is made between a measured value of the specific heat of the evaluation object ceramics and a known value of the specific heat of ceramics to be a reference at that predetermined temperature. By this comparison, mechanical properties of the evaluation object ceramics are presumed from known mechanical properties of the reference ceramics.

In accordance with a method of evaluating ceramics according to another aspect of the present invention, a specific heat of each of a plurality of ceramics to be references is measured at a predetermined temperature. Also, mechanical properties of each of those reference ceramics are measured. A first correlation between the measured specific heats and the measured mechanical properties is determined. Further, a specific heat of ceramics being an object of evaluation is measured at a predetermined temperature. By use of the above-described correlation, mechanical properties of the evaluation object ceramics are obtained from a measured value of the specific heat of the evaluation object ceramics.

In accordance with the method of evaluating ceramics according to the present invention, a specific heat of each of a plurality of ceramics to be references is measured at a predetermined temperature. Also, the amount of glass phase included in each of those reference ceramics is measured. A second correlation between the measured specific heats and the measured amount of glass phase is determined. Further, a specific heat of ceramics to be an object of evaluation is measured at a predetermined temperature. By use of the above-described correlation, the amount of glass phase included in the ceramics to be the evaluation object is obtained from a measured value of the specific heat of the evaluation object ceramics.

The inventors of the present application focused on the fact that a specific heat of glass phase or the like formed of such oxide as of aluminum or yttrium, which is to be added as a sintering aid, is large in a low temperature region, and they made various considerations. Consequently, they discovered that by measuring and comparing specific heats of ceramics at temperatures not higher than room temperature, they were able to make a precise comparison in amounts of glass phase or the like included in the ceramics and further a comparison in mechanical properties of the ceramics. The present invention was made on the basis of such discovery and knowledge of the inventors.

That is, a specific heat of a substance changes, in general, in accordance with temperature. However, while crystallized ceramics such as silicon nitride has a small specific heat at a low temperature, the glass phase or the like formed of the above-described oxide has a large specific heat in a low temperature region. Thus, the specific heat of ceramics such as a silicon nitride sintered body including glass phase or the like is in correlation with the amount of glass phase or the like included therein in the low temperature region in which the specific heat of glass phase or the like is larger than that of crystal phase. In addition, even if the amount of glass phase or the like makes only a slight change, the specific heat of corresponding ceramics makes a substantially large change. The low temperature region, in which the above relation between temperature and specific heat is obtained, is a temperature range not higher than room temperature, i.e., a range between room temperature and absolute zero degree. The discovery and knowledge as above was obtained by the inventors of the present application.

Accordingly, if silicon nitride is taken as an example, the specific heat of a certain silicon nitride sintered body is measured at a temperature not higher than room temperature by a normal method by utilizing a normal freezing mixture or a cryostat apparatus (low temperature thermostat) employing liquid nitrogen, liquid helium or the like. Independently of this measurement of specific heat, the specific heat (a reference value) of a reference silicon nitride sintered body, the amount of glass phase or the like of which is known by the structure observation method or the like or which has predetermined mechanical properties, is measured in advance at the same temperature as the above temperature not higher than room temperature. By comparing this specific heat (reference value) and the specific heat (measured value) of the above-described silicon nitride sintered body being the evaluation object, it becomes possible to relatively evaluate the amount of glass phase or the like or the mechanical properties of the silicon nitride sintered body being the evaluation object.

Moreover, the amount of glass phase or the like or mechanical properties, and specific heats at a temperature not higher than room temperature are measured, respectively, with respect to a large number of silicon nitride sintered bodies to be references. By plotting in advance in a graph the correlation between the measured amount of glass phase or the like or the measured mechanical properties and the measured specific heats, correlation curves are obtained. On the other hand, the specific heat of the silicon nitride sintered body being the evaluation object at the same temperature as above is measured. It is possible to obtain as absolute values the amount of glass phase or the mechanical properties of that sintered body from a measured value of its specific heat by employing the above-described correlation curves.

The ceramics evaluation method of the present invention is also applicable to such various types of ceramics as aluminum nitride, silicon carbide, aluminum oxide, zirconium oxide or mullite other than the ceramics of silicon nitride.

As described above, according to the ceramics evaluation method of the present invention, since there is no need to destroy or process ceramics (sample) as the object of evaluation, not a sampling inspection but an inspection on a nondestructive production line is available. This makes it possible to guarantee the amount of glass phase or the like or mechanical properties with respect to ceramics which are actually supplied as products or the like. Further, the evaluation by the method of the present invention is information as to all of individual ceramics, not partial information limited to a specific part of ceramics as by the X-ray diffraction method.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a graph showing a relationship between a specific heat and a creep strength or the amount of glass phase of a silicon nitride sintered body as reference ceramics obtained in Example 1.

Fig. 2 is a graph showing a relationship between respective specific heats of silicon nitride sintered bodies including different amounts of glass phase included therein obtained in Example 2, and temperatures.

### DESCRIPTION OF THE PREFERRED EMBODIMENTS

### Example 1

Si₃N₄ powder of 0.8µm in average grain size and 1.5% by weight in oxygen content, Y₂O₃ powder, Al₂O₃ powder and SiO₂ powder were prepared. Those powder were mixed together to form such compositions as shown in Table 1 below, and the respective samples were sintered under respective sintering conditions shown in Table 1. Sample No. 1 consisting of the Si₃N₄ powder was, however, subjected to HIP sintering by a glass capsule method.

**Table 1**

| Sample | Composition of sintered body | | | | Sintering Condition |
|---|---|---|---|---|---|
| Number | Si₃N₄ | Y₂O₃ | Al₂O₃ | SiO₂ | (°C x hr) |
| 1 | 100 | - | - | - | 1750 x 2 |
| 2 | 96 | 3 | 1 | - | 1750 x 2 |
| 3 | 92 | 5 | 3 | - | 1750 x 2 |
| 4 | 87 | 5 | 3 | 5 | 1750 x 2 |
| 5 | - | 61 | 28 | 11 | 1600 x 1 |

Each of the resultant sintered bodies was put into a cryostat apparatus, their specific heats were measured in a temperature range of -271°C to room temperature by using a calorimeter, and specific heats at -268°C (5K) were obtained. Then, the amount of phase or the like included in each sintered body was measured, and a creep strength at 1350°C x 500hr and fracture toughness of each sintered body was measured. The result is shown in Table 2 below.

**Table 2**

| Sample | Glass phase | Specific heat | Creep | Fracture toughness |
|---|---|---|---|---|
| Number | (mol%) | (J/K·Kg) | Strength (MPa) | (MN/m^{3/2}) |
| 1 | 0 | 0.1825 | 300 | 4 |
| 2 | 5 | 0.1910 | 250 | 6 |
| 3 | 10 | 0.2050 | 200 | 7 |
| 4 | 20 | 0.2310 | 150 | 6 |
| 5 | 100 | 0.3200 | 10 | 1 |

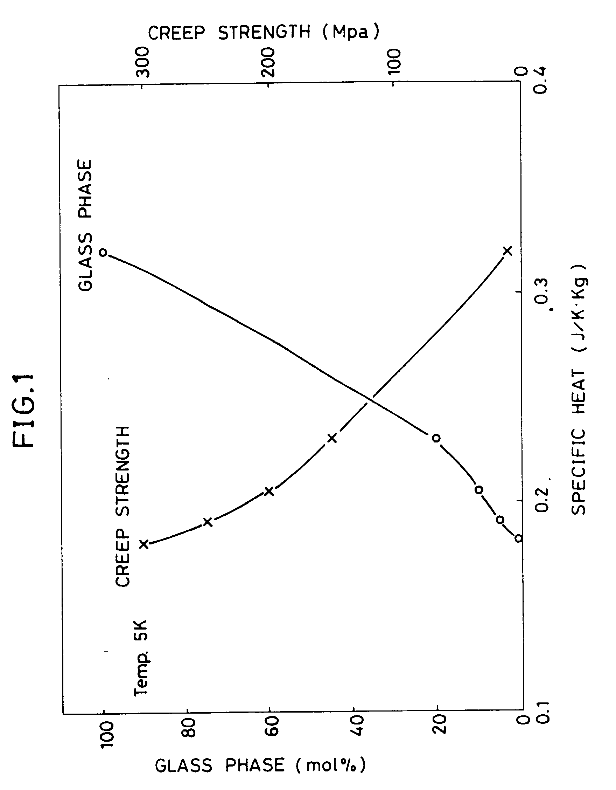

A relationship between the amount of glass phase or the creep strength and the specific heat (of each sample), which was made based on Table 2, is shown in Fig. 1. Curves shown in Fig. 1 are indicative of a correlation between the creep strength or the amount of glass phase and the specific heat of ceramics to be references. As apparent from Fig. 1, it is understood that a sintered body having a larger specific heat has a larger amount of glass phase or the like included therein and a smaller creep strength. This makes it possible to measure the specific heat of a silicon nitride sintered body being the object of evaluation and also relatively compare and evaluate the amount of glass phase or the like and further the creep strength as one of mechanical properties from the measured value of specific heat by employing the correlation curves of Fig. 1.

### Example 2

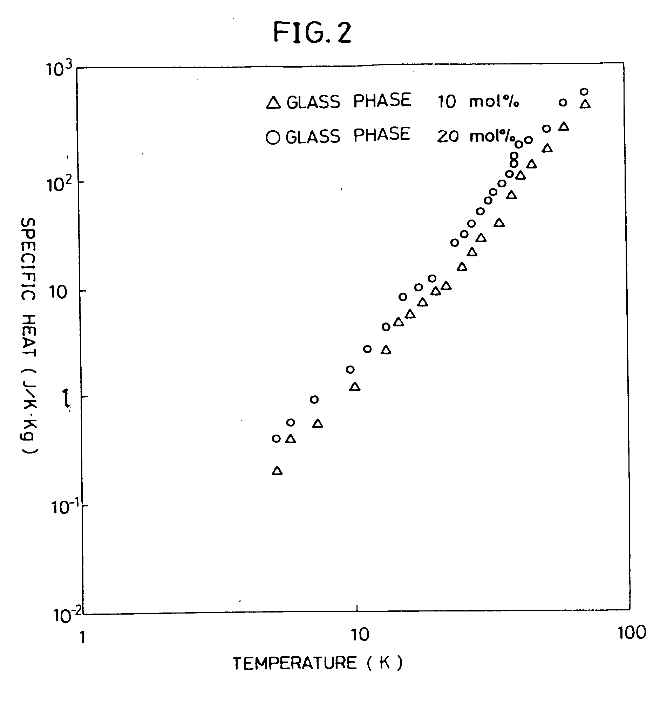

Y₂O₃ powder and Al₂O₃ powder were added to and mixed with Si₃N₄ powder of 0.5µm in average grain size and 2.0% by weight in oxygen content. The mixture was then sintered, and two types of silicon nitride sintered bodies were produced: the one includes 10 mol% of glass phase, and the other includes 20 mol% of glass phase. Respective specific heats of the resultant two sintered bodies were measured in a temperature range of 5K to 76K. Relationships between the specific heats based on the result of measurement and temperature are shown in Fig. 2. As apparent from Fig. 2, it is recognized that there is a difference in specific heat due to a difference in amount of glass phase in a low temperature region not higher than room temperature.

As has been described above, according to the present invention, it is possible to evaluate ceramics such as silicon nitride sintered bodies by a nondestructive inspection introduced into a production line and guarantee the amount of glass phase or the like or mechanical properties with respect to individual ceramics which are supplied as products or the like in practice.

Although the present invention has been described and illustrated in detail, it is clearly understood that the same is by way of illustration and example only and is not to be taken by way of limitation, the scope of the present invention being limited only by the terms of the appended claims.

## Claims

1. A method of evaluating ceramics, comprising steps of:
measuring a specific heat of each of a plurality of reference ceramics at a predetermined temperature;
measuring mechanical properties of each of said plurality of reference ceramics;
measuring an amount of glass phase included in each of said plurality of reference ceramics;
determining a first correlation between said specific heat and said mechanical properties and a second correlation between said specific heat and said amount of glass phase;
measuring a specific heat of ceramics being an object of evaluation at a predetermined temperature;
obtaining mechanical properties and the amount of glass phase of said evaluation object ceramics from a measured value of the specific heat of said evaluation object ceramics by using the first correlation and the second correlation, respectively.

2. Method according to claim 1, **characterized in that** said ceramics comprise a silicon nitride sintered body.

3. Method according to claim 1 or 2, **characterized in that** said predetermined temperature is a temperature lower than or equal to room temperature.

4. Method according to claims 1 to 3, **characterized in that** said mechanical properties comprise a creep strength.

## Patentansprüche

1. Verfahren zur Bewertung von Keramiken, **gekennzeichnet durch** die Schritte:
Messen einer spezifischen Wärmekapazität von jeweils einer von mehreren Referenzkeramiken bei einer vorgegebenen Temperatur;
Messen von mechanischen Eigenschaften von jeweils einer der mehreren Referenzkeramiken;
Messen eines Anteils an Glasphase, der in jeweils einer der mehreren Referenzkeramiken enthalten ist;
Ermitteln einer ersten Korrelation zwischen der spezifischen Wärmekapazität und den mechanischen Eigenschaften und einer zweiten Korrelation zwischen der spezifischen Wärmekapazität und dem Anteil an Glasphase;
Messen einer spezifischen Wärmekapazität einer Keramik, die Gegenstand der Bewertung bei einer vorgegebenen Temperatur ist;
Erhalten von mechanischen Eigenschaften und dem Anteil an Glasphase in der zu bewertenden Keramik aus einem gemessenen Wert der spezifischen Wärmekapazität der zu bewertenden Keramik durch die Verwendung der ersten Korrelation bzw. der zweiten Korrelation.

2. Verfahren nach Anspruch 1, **dadurch gekennzeichnet, daß** die Keramik einen gesinterten Körper aus Siliziumnitrid aufweist.

3. Verfahren nach Anspruch 1 oder 2, **dadurch gekennzeichnet, daß** die vorgegebene Temperatur kleiner oder gleich der Raumtemperatur ist.

4. Verfahren nach einem der Ansprüche 1 bis 3, **dadurch gekennzeichnet, daß** die mechanischen Eigenschaften eine Dauerdehngrenze umfassen.

## Revendications

1. Procédé d'évaluation d'une céramique, comprenant les étapes consistant à :
mesurer une chaleur massique de chaque céramique de référence parmi une pluralité de celles-ci à une température prédéterminée ;
mesurer les propriétés mécaniques de chacune desdites plusieurs céramiques de référence ;
mesurer une proportion de phase cristalline comprise dans chacune desdites plusieurs céramiques de référence ;
déterminer une première relation entre ladite chaleur massique et lesdites propriétés mécaniques et une seconde relation entre ladite chaleur massique et ladite proportion de phase cristalline ;
mesurer une chaleur massique de la céramique qui est l'objet de l'évaluation à une température prédéterminée ;
obtenir les propriétés mécaniques et la proportion de phase cristalline de ladite céramique qui est l'objet de l'évaluation à partir d'une valeur mesurée de la chaleur massique de ladite céramique qui est l'objet de l'évaluation en utilisant respectivement la première relation et la seconde relation.

2. Procédé selon la revendication 1, caractérisé en ce que ladite céramique comprend un corps fritté au nitrure de silicium.

3. Procédé selon la revendication 1 ou 2, caractérisé en ce que ladite température prédéterminée est une température inférieure ou égale à la température ambiante.

4. Procédé selon les revendications 1 à 3, caractérisé en ce que lesdites propriétés mécaniques comprennent une résistance au fluage.
